# EUROPEAN PATENT SPECIFICATION

(11) **EP 3 278 823 B1**
(45) Date of publication and mention of the grant of the patent: **15.09.2021**
(21) Application number: 16182832.2
(22) Date of filing: 04.08.2016
(51) Int. Cl.: A61M 5/142, A61M 5/168, A61M 5/172

(54) **DEVICE FOR MONITORING THE FLOW OF A FLUID IN A FLUID ADMINISTRATION SYSTEM**
VORRICHTUNG ZUR ÜBERWACHUNG DES FLUSSES EINER FLÜSSIGKEIT IN EINEM FLÜSSIGKEITSVERABREICHUNGSSYSTEM
DISPOSITIF DE SURVEILLANCE DE L'ÉCOULEMENT D'UN FLUIDE DANS UN SYSTÈME D'ADMINISTRATION DE FLUIDE

(43) Date of publication of application: 07.02.2018
(73) Proprietor: Roche Diabetes Care GmbH, 68305 Mannheim (DE); F. Hoffmann-La Roche AG, 4070 Basel (CH)
(72) Inventor: LAMBERTSON, Michael, 68305 Mannheim (DE); WUERTELE, Christian, 68163 Mannheim (DE)
(74) Representative: Rentsch Partner AG

(56) References cited:
- EP-A1- 2 857 803
- WO-A1-2014/016316
- US-A1- 2007 034 016
- US-B1- 6 475 170
- None

## Description

### FIELD OF THE INVENTION

The present invention relates to a device for monitoring the flow of a fluid in a fluid administration system.

### BACKGROUND ART

In fluid administration systems, such as insulin administration systems comprising an insulin pump for administering insulin to a patient, precise monitoring of the flow of the fluid is desired. When monitoring the flow of fluid, it is desired to detect if the fluid is flowing properly, if an occlusion occurred, if the fluid contains bubbles, if a pump reservoir is leaking fluid, if infusion sets come loose, etc.

In order to monitor the flow of fluid in a fluid administration system, a flow chamber having installed a piezoelectric sensor can be used. The flow chamber can be designed such that an amount of the fluid of the fluid administration system flows into the flow chamber and transmits a mechanical force to the piezoelectric sensor. For example, on activation of a pump installed in the fluid administration system, an increased pressure in the fluid can be monitored with the piezoelectric sensor. However, the piezoelectric sensor registers also other mechanical interferences, such as vibrations because of movements of the patient, for example when the patient makes sport exercises, because of shocks such as of a pump falling on a hard surface, etc. Because of such mechanical interferences, it is difficult to decompose the signal recorded with the piezoelectric sensor and to monitor the flow of the fluid in the fluid administration system sufficiently precise that it can be determined if the fluid is flowing properly, if an occlusion has occurred, etc.

WO9603168A1 relates to monitoring pressure conditions upstream and downstream in an intravenous fluid administration system and detecting upstream and/or downstream occlusions. Signal changes in fluid pressure are detected across the wall of the tubing so that no interruption in the fluid tubing is required. The mechanical sensors monitor relative pressure conditions. A normalization process accounts for changes in the normal tube force and/or tube resiliency.

US2009177148A1 relates to detecting an inclusion within an infusion system. An output signal of a flowrate sensor is monitored as fluid flows through the infusion system. The output signal is converted with a noise reduction or averaging filter to obtain a filtered output signal. An alarm is activated if the filtered signal falls below a desired threshold, thereby indicating an occlusion within the infusion system. In an embodiment, the signal is filtered with a backward averaging filter. Converting the output signal with the noise reduction filter suppresses the peaks and valleys of the signal and also suppresses patient induced artefacts. The risk of false alarms is reduced.

EP2601884A1 discloses an active noise cancellation device for a medical device. An active circuit has a first input connection, a second input connection, and an output connection. The second input connection is connected to a predetermined reference signal. The active noise cancellation device further includes a low-impedance body connection electrode adapted to be in electrical contact with a bloodstream of a subject. The low-impedance body connection is connected to the first input connection. A feedback branch connects the output connection with the first input connection. The feedback branch comprises a current limiting circuit to limit a current through the feedback branch to be lower than a predetermined current. The predetermined reference signal can be any stable voltage such as a fixed DC reference voltage related to a ground of the medical device.

US7448382 discloses a medical device, such as a respiratory treatment device, having an active acoustic noise cancellation system. A detector detects characteristics of acoustic noise of a blower assembly. A speaker creates a cancelation frequency in the form of acoustic waves that at least partially cancels acoustic waves generated by the blower assembly. In another embodiment, a mechanical vibration generating element creates a cancellation frequency in the form of a mechanical vibration that at least partially cancels vibrations of the blower assembly.

WO 2014/016316 A1 discloses a flow sensor according to the state of the art.

### DISCLOSURE OF THE INVENTION

It is an object of the present invention to provide a device for monitoring the flow of fluid in a fluid administration system, which device do not have at least some of the disadvantages of the prior art. In particular, it is an object of the present invention to provide a device for monitoring the flow of a fluid in a fluid administration system, which device enables precise monitoring of the flow of fluid in the fluid administration system also in case the fluid administration system is exposed to a wide variety of noise sources, such as mechanical noise sources because of movements of the patient, shocks because of a pump falling on a hard surface, etc.

According to the present invention, these objects are achieved through the features of the independent claims. In addition, further advantageous embodiments follow from the dependent claims and the description.

According to the present invention, the above-mentioned objects are particularly achieved by a flow sensor for enabling determination of a flow signal corresponding to a flow of a fluid of a fluid administration system. The flow sensor comprises a housing. The housing has arranged a flow input, a flow chamber and a flow output being designed that the flow input and the flow output can be connected to the fluid administration system such that at least an amount of the fluid of the fluid administration system can consecutively pass the flow input, the flow chamber and the flow output. The housing has further arranged a first mechanical sensor for recording a raw flow signal corresponding to the flow of the fluid passing the flow chamber and an ambient noise. The housing has further arranged a second mechanical sensor for recording an ambient noise signal corresponding to the ambient noise. The housing and the arrangement of the first mechanical sensor and the arrangement of the second mechanical sensor are designed that a signal processing unit receiving the raw flow signal and the ambient noise signal is enabled to determine the flow signal from the raw flow signal and the ambient noise signal in such a manner that the flow signal exhibits less ambient noise than the raw flow signal. The arrangement of the first mechanical sensor is sufficiently close to the fluid of the fluid administration system and the arrangement of the second mechanical sensor is sufficiently distant from the fluid of the fluid administration system such that the first mechanical sensor records the superposition of the flow of the fluid and the ambient noise and the second mechanical sensor records the ambient noise, which enables a signal processing unit to determine the flow signal by cancelling ambient noise in the raw flow signal.

In an embodiment, the first mechanical sensor is arranged at a first location of the housing and the second mechanical sensor is arranged at a second location of the housing, preferably the first mechanical sensor and the second mechanical sensor are arranged at opposite sides of the housing.

The first mechanical sensor is arranged closer to the flow chamber than the second mechanical sensor.

In an embodiment, the flow sensor is designed such that the amount of fluid is essentially in direct contact with the first mechanical sensor, wherein preferably a thin membrane is arranged for sterilization purposes.

In an embodiment, the second mechanical sensor is arranged at a location of the housing providing an open chamber between the second mechanical sensor and the housing.

In an embodiment, the flow sensor is designed such that at least an amount of fluid flows at least once essentially vertically towards the first mechanical sensor, at least once essentially vertically away from the first mechanical sensor, and/or essentially parallel along the first mechanical sensor.

In an embodiment, the flow sensor is designed such that at least one of width, height, and diameter of at least an amount of fluid flowing through the flow sensor is kept essentially constant.

In an embodiment, the flow chamber has essentially the form of a cylinder, the cylinder preferably having a height being defined in the direction of a surface of the housing which is smaller than a diameter of the cylinder, or wherein the flow chamber has essentially the form of a cuboid, the cuboid preferably having a height being defined in the direction of a surface of the housing which is smaller than a width of the cuboid and/or a length of the cuboid.

In an embodiment, the first mechanical sensor and/or the second mechanical sensor have essentially a disc-shaped form.

In an embodiment, the first mechanical sensor and/or the second mechanical sensor have essentially a rectangular form.

In an embodiment, the first mechanical sensor and/or the second mechanical sensor includes a piezoelectric element, preferably a piezoelectric buzzer.

In an embodiment, the flow sensor is mounted in a patch pump, a durable pump, an adapter, or an infusion head.

In an embodiment, the flow sensor is connected to a signal processing unit, the signal processing unit having a first input for receiving the raw flow signal from the first mechanical sensor, and a second input for receiving the ambient noise signal from the second mechanical sensor, the signal processing unit being enabled to determine from the raw flow signal and the ambient noise signal the flow signal in such a manner that the flow signal exhibits less ambient noise than the raw flow signal.

In an embodiment, the signal processing unit is enabled to subtract the ambient noise signal from the raw flow signal.

In an embodiment, the signal processing unit is enabled compare a rise time and/or a fall time of the flow signal and/or an amplitude of several peaks in the flow signal to a predetermined threshold in order to detect if the fluid administrating system does not or does include a blockage, in particular an occlusion.

### BRIEF DESCRIPTION OF THE DRAWINGS

The herein described invention will be more fully understood from the detailed description given herein below and the accompanying drawings which should not be considered limiting to the invention described in the appended claims. The drawings are showing:
- Fig. 1a: illustrates schematically a side view of a flow sensor in accordance to the invention;
- Fig. 1b: illustrates schematically a top view of a flow sensor in accordance to the invention;
- Fig. 2: illustrates schematically a signal processing unit;
- Fig. 3: illustrates an embodiment of the flow sensor;
- Fig. 4: illustrates an embodiment of the flow sensor;
- Fig. 5: illustrates schematically a patch pump and a flow sensor;
- Fig. 6: illustrates schematically a durable pump and an infusion head and a flow sensor;
- Fig. 7: illustrates a flow sensor which is arranged between fluidic tubings providing the fluid from a pump and delivering the fluid to an infusion head;
- Fig. 8a: illustrates the so called "forcesensor technology" widely used in prior art;
- Fig. 8b: illustrates the so called "forcesensor technology" widely used in prior art;
- Fig. 9: illustrates schematically a signal that was recorded by a single mechanical sensor;
- Fig. 10: illustrates schematically the flow signal which has been determined by a signal processing unit receiving a raw flow signal (not shown in Figure 10) and the ambient noise signal;
- Fig. 11: illustrates schematically a sequence of motor bursts;
- Fig. 12: illustrates schematically an electronic circuit diagram of a signal processing unit;
- Fig. 13: illustrates schematically a container for storing a medical or pharmaceutical liquid; and
- Fig. 14: illustrates schematically sequences of motor bursts.

### MODE(S) FOR CARRYING OUT THE INVENTION

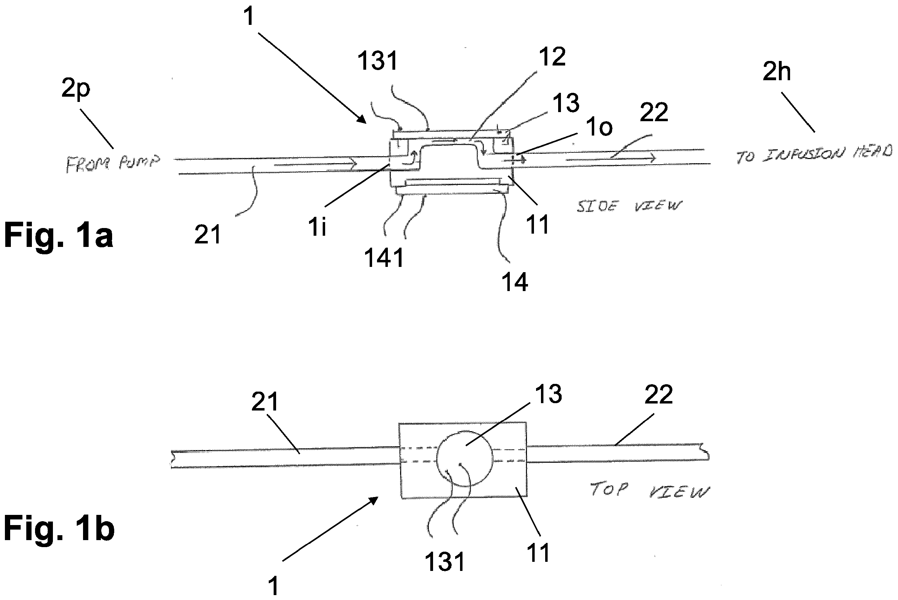

Figure 1a illustrates schematically a side view of a flow sensor 1. Figure 1b illustrates schematically a top view of the flow sensor 1. The flow sensor 1 has a housing 11. As illustrated in Figure 1a and Figure 1b, the housing 11 can have the form of a cuboid. The housing 11 has arranged a flow input 1i, a flow chamber 12 and a flow output 1o. As illustrated in Figure 1a and Figure 1b, the flow input 1i and the flow output 1o can be connected to a fluid administration system.

The fluid administration system is designed to administer a fluid to a patient. As illustrated in Figure 1a, the fluid administration system comprises a fluid pump 2p, fluidic tubings 21, 22 and an infusion head 2i. The fluid is delivered from the fluid pump 2p to a first fluidic tubings 21. The fluid is delivered from the second fluidic tubing 22 to the infusion head 2h for administering the fluid to the patient. Figure 1a and Figure 1b illustrate schematically parts of the fluid administration system, the parts comprising the fluid pump 2p, the fluidic tubings 21, 22, and the infusion head 2i. However, the fluid administration system can comprise further parts.

As illustrated in Figure 1a, the fluid pump 2p is connected to the first tubing 21. As illustrated in Figure 1a and Figure 1b, the first tubing is connected to the flow input 1i of the housing 11 of the flow sensor 1. As illustrated in Figure 1a, the flow output 1o of the housing 11 of the flow sensor 1 is connected to the second tubing 22. As illustrated in Figure 1a and Figure 1b, the second fluidic tubing 22 is connected to the infusion head 2h.

The housing 11 of the flow sensor 1 has arranged the flow input 1i, the flow chamber 12 and the flow output 1o such that the fluid of the fluid administration system can consecutively pass the flow input 1i, the flow chamber 12 and the flow output 1o. Accordingly, the fluid pump 2p pumps fluid into the first fluidic tubing 21. The fluid passes the first fluidic tubing 21 and flows into the fluid input 1i of the housing 11. The fluid passes the flow chamber 12 and the flow output 1o. The fluid flows from the fluid output 1o into the second fluidic tubing 22 and passes the second fluidic tubing 22. The fluid flows into the infusion head 2h and from the infusion head 2h into the patient. Accordingly, the fluid can be administered from the fluid pump 2p into the patient.

The flow of the fluid is illustrated in Figure 1a by arrows. The fluid flows from the pump 2p through the first fludic tubing 21 into the flow input 1i of the housing 11 of the flow sensor 1. In the housing 11, the fluid turns upwards after the fluid input 1i, then turns into a horizontal direction in the region of the fluid chamber 12, then turns downwards again before leaving the housing 11 through the flow output 2o. The directions upwards, horizontal, downwards, etc. are defined with respect to the arrangement of the flow sensor 1 according to Figure 1a and Figure 1b.

As illustrated in Figure 1a and Figure 1b, the housing 11 of the flow sensor 1 has arranged a first mechanical sensor 13 for recording a raw flow signal corresponding to the flow of the fluid passing the flow chamber 12. For example, the first mechanical sensor 13 can record a raw flow signal that corresponds to changes in the pressure of the fluid.

As illustrated in Figure 1a, the first mechanical sensor 13 is arranged on the top of the housing 11 of the flow sensor 1. The first mechanical sensor 13 can form a side wall of the flow chamber 12. A thin membrane can be arranged between the first mechanical sensor 13 and the fluid flowing in the flow chamber 12 for sterilization purposes.

As illustrated in Figure 1a and Figure 1b, the first mechanical sensor 13 has attached electrical connections 131 for transmitting a raw flow signal recorded with the mechanical sensor 13 to a signal processing unit (not shown in Figure 1a and Figure 1b). The raw flow signal recorded with the first mechanical sensor 13 corresponds to the flow of the fluid passing the flow chamber 12.

As illustrated in Figure 1a and Figure 1b, the housing 11 of the flow sensor 1 has arranged a second mechanical sensor 14 for recording an ambient noise signal corresponding to an ambient noise. For example, the second mechanical sensor 14 can record an ambient noise signal that corresponds to movements of the patient, shocks because of an interaction with a hard surface, etc.

As illustrated in Figure 1a, the second mechanical sensor 14 is arranged on the bottom of the housing 11 of the flow sensor 1. As illustrated in Figure 1a, the housing 11 includes a separation element 111 forming a separation between the flow chamber 12 and the second mechanical sensor 14. The second mechanical sensor 14 can be arranged at the bottom wall of the housing 11.

For example, the first mechanical sensor 13 and the second mechanical sensor 14 can each comprise a piezoelectric element which transforms in each case a mechanical movement into an electrical signal.

The first mechanical sensor 13 can sense mechanical movements induced by a change in the pressure of fluid flowing in the fluid chamber 12. At the same time, the first mechanical sensor 13 may also sense mechanical movements induced by an ambient noise. Accordingly, the electrical signal of the first mechanical sensor 13 is a raw flow signal corresponding to the flow of the fluid passing the flow chamber and to an ambient noise.

The second mechanical sensor 14 is arranged such that mechanical movements induced by a change in the pressure of fluid flowing in the fluid chamber 12 cannot be sensed, in particular because the second mechanical sensor 14 is separated from the fluid chamber 12, for example, by the separation element 111. However, the second mechanical sensor 14 may sense mechanical movements induced by an ambient noise. Accordingly, the electrical signal of the second mechanical sensor 13 is an ambient noise signal corresponding to an ambient noise signal.

In particular, the second mechanical sensor 14 may sense the same or similar mechanical movements induced by an ambient noise as are sensed by the first mechanical sensor 13.

The electrical connections 131 of the first mechanical sensor 13 and the electrical connections 141 of the second mechanical sensor 14 are connected to a signal processing unit (not shown in Figure 1a and Figure 1b). The signal processing unit is enabled to determine a flow signal from the raw flow signal of the first mechanical sensor 13 and the ambient noise signal of the second mechanical sensor 14 in such a manner that the flow signal determined by the signal processing unit exhibits less noise than the raw flow signal.

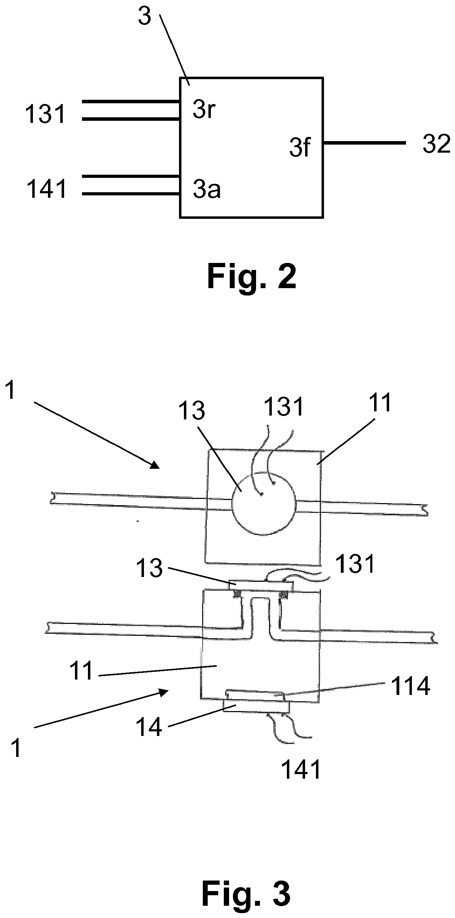

Figure 2 illustrates schematically a signal processing unit 3 which is connected to the electrical connections 131 of the first mechanical sensor 13 (not shown in Figure 2) and the electrical connections 141 of the second mechanical sensor 14 (not shown in Figure 2). The signal processing unit 3 receives from the electrical connections 131 of the first mechanical sensor 13 the raw flow signal 3r. The signal processing unit 3 receives from the electrical connections 141 of the second mechanical sensor 14 the ambient signal 3a. The signal processing unit 3 determines from the raw flow signal 3r and the ambient noise signal 3a a flow signal 3f that exhibits less ambient noise than the raw flow signal 3r. For example, the flow signal 3f is determined by subtracting the ambient noise signal 3a from the raw flow signal 3r.

The first mechanical sensor 13 is arranged at a first location of the housing 11. As illustrated in Figure 1a, the first mechanical sensor 13 is arranged at the top of the housing 11. The second mechanical sensor 14 is arranged at a second location of the housing 11. As illustrated in Figure 1a, the second mechanical sensor 14 is arrange at the bottom of the housing 11. As illustrated in Figure 1a, the first mechanical sensor 13 and the second mechanical sensor 14 are arranged at opposite sides of the housing 11.

As illustrated in Figure 1a, the first mechanical sensor 13 is arranged closer to the flow chamber 12 than the second mechanical sensor 14.

As illustrated in Figure 1a, the first mechanical sensor 13 is essentially in direct contact with the first mechanical sensor 13. For sterilization purposes, a thin membrane can be arranged.

Figure 3 illustrates an embodiment of the flow sensor 1 in a side view (above) and in a top view (below). As illustrated in Figure 3, the first mechanical sensor 13 has a round form and is arranged at the top of the housing 11. The second mechanical sensor 14 is arranged at the bottom of the housing 11. The first mechanical sensor 13 has electrical connections 131. The second mechanical sensor 14 has electrical connections 141.

As illustrated in Figure 3, a recess is formed at the housing 11 in order to provide an open chamber 114 between the second mechanical sensor 14 and the housing 11. The open chamber 114 allows for free movement of elements, such as the piezo, of the second mechanical sensor 14 for ambient noise detection.

As illustrated in Figure 1a, an open chamber (no reference sign shown in Figure 1a) can be formed between the housing 11 and the second mechanical sensor 4 allowing for free movement of elements, such as the piezo, of the second mechanical sensor 14 for ambient noise detection.

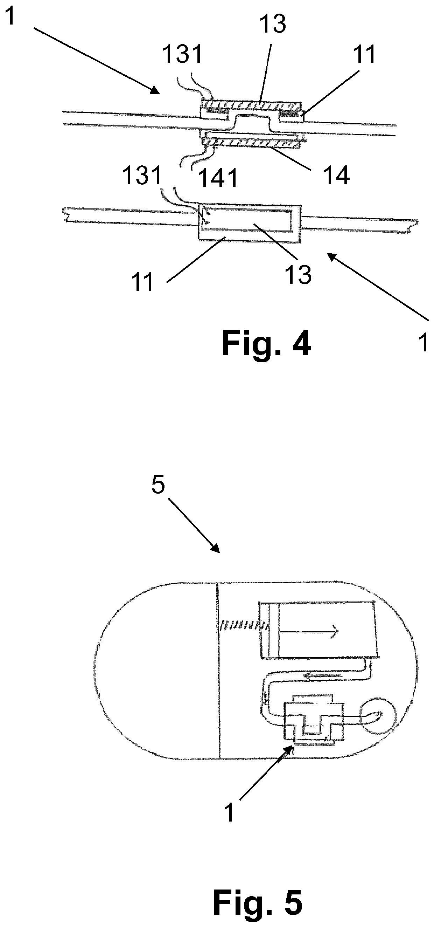

Figure 4 illustrates an embodiment of the flow sensor 1 in a side view (above) and in a top view (below). As illustrated in Figure 4, the first mechanical sensor 13 has a rectangular form and is arranged at the top of the housing 11. The second mechanical sensor 14 is arranged at the bottom of the housing 11. The first mechanical sensor 13 has electrical connections 131. The second mechanical sensor 14 has electrical connections 141. An open chamber (no reference sign shown in Figure 4) can be formed between the housing 11 and the second mechanical sensor 4 allowing for free movement of elements, such as the piezo, of the second mechanical sensor 14 for ambient noise detection.

As illustrated in Figure 1a, Figure 3 and Figure 4, the flow sensor 1 is designed such that at least an amount of fluid flows at least once essentially vertically towards the first mechanical sensor 13, at least once essentially vertically away from the first mechanical sensor 13, and/or essentially parallel along the first mechanical sensor 13.

The fluid flowing through the flow sensor 1 can be defined by its width, a height, and/or diameter. The width of the fluid is defined in accordance to the Figures showing a top view of the flow sensor 1. The height of the fluid is defined in accordance to the Figures showing a side view of the flow sensor 1. The diameter of the fluid is defined in case the fluid flows in a tubing having a disc-shaped cross section. The width of the fluid is defined in case the fluid flows in a tubing having a quadratic or rectangular cross section. In the flow sensors 1 shown according to Figure 1a, Figure 3 and Figure 4, the fluid flows in a form having disc-shape cross section, a quadratic cross section, a rectangular cross section or a combination thereof. The flow chamber 12 can have a form that corresponds to the first mechanical sensor 13. The flow chamber 12 of the flow sensor 1 illustrated in Figure 1a, 1b can have the form of a hollow cylinder. The flow chamber 12 of the flow sensor 1 illustrated in Figure 3 can have the form of a hollow cylinder. The flow chamber 12 of the flow sensor 1 illustrated in Figure 4 can have the form of a hollow cuboid.

As illustrated in Figure 3, the flow chamber 12 can have an essentially cylindrical form, wherein a height which is defined in the direction of a surface of the housing 11, namely in the direction of the first mechanical sensor 13, is smaller than a diameter.

As illustrated in Figure 4, the flow chamber 12 can have an essentially cuboid form, wherein a height which is defined in the direction of a surface of the housing 11, namely in the direction of the first mechanical sensor 13, is smaller than a width of the cuboid and/or a length of the cuboid.

As illustrated in Figure 3 and Figure 4, the first mechanical sensor 13 and/or the second mechanical sensor 14 can have an essentially disc-shaped form.

As illustrated in Figure 3 and Figure 4, the first mechanical sensor 13 and/or the second mechanical sensor 14 can have an essentially rectangular form.

The first mechanical sensor 13 and/or the second mechanical sensor 14 can include a piezoelectric element, preferably a piezoelectric buzzer, which is cheap and available off the shelf.

Figure 5 illustrates schematically a patch pump 5. The patch pump 5 can have mounted a flow sensor 1. The flow sensor 1 can be mounted in the disposable part of the patch pump 5.

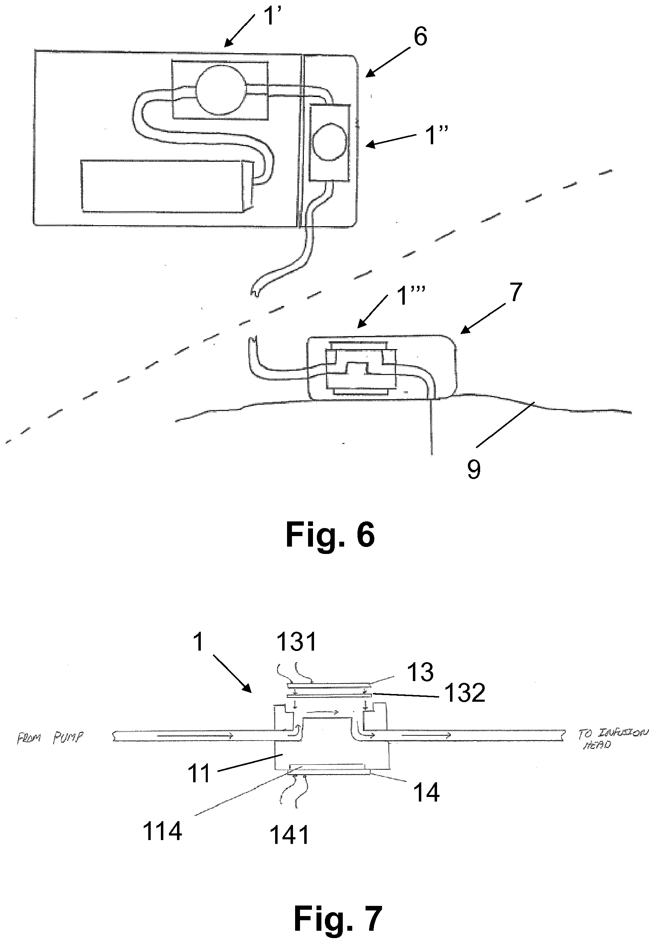

Figure 6 illustrates schematically a durable pump 6 and an infusion head 7. The durable pump 6 is located at a different location than the infusion head 7. The infusion head 7 is attached to the skin 9 of a patient in order to administer the fluid.

As illustrated in Figure 6, a flow sensor 1 can be mounted at two possible positions of the durable pump. The flow sensor with reference sign 1' is mounted in the durable part of the durable pump 6. The flow sensor with reference sign 1" is mounted in the adapter and tubing of the durable pump 6.

As illustrated in Figure 6, a flow sensor 1 can be mounted in the infusion head 7. The flow sensor with reference sign 1''' is mounted in the infusion head 7.

One or more of the flow sensors 1', 1", 1''' can be mounted in the durable pump 6 and/or the infusion head 7. Figure 6 illustrates the case where all flow sensors 1', 1", 1''' are mounted. However, only one of the flow sensors 1', 1", 1''' can be mounted.

Figure 7 illustrates a flow sensor 1 which is arranged between fluidic tubings providing the fluid from a pump and delivering the fluid to an infusion head. The flow sensor 1 has a housing 11 with a recess at the top in the form of a cylinder or cuboid. As illustrated with arrows in Figure 7, a thin membrane 132 is arranged in the recess for sterilization purposes prior to arranging the first mechanical sensor 13. The thin membrane 132 is arranged for sterilization purposes. In particular, the thin membrane 132 has been sterilized. At least relevant parts of the housing 11, the fluidic tubings, the infusion pump and the infusion head are sterilized as well. Therefore, the fluid flowing from the infusion pump to the infusion head does not come into contact with parts that are not sterilized. In particular, when passing the flow chamber 12, the fluid does not come into contact with the first mechanical sensor 13, which may be difficult to be sterilized.

As illustrated in Figure 7, the housing 11 includes a recess at the location where the second mechanical sensor 14 is installed. Accordingly, an open chamber 114 is formed. The open chamber 114 allows for free movement of elements, such as the piezo, of the second mechanical sensor 14 for ambient noise detection.

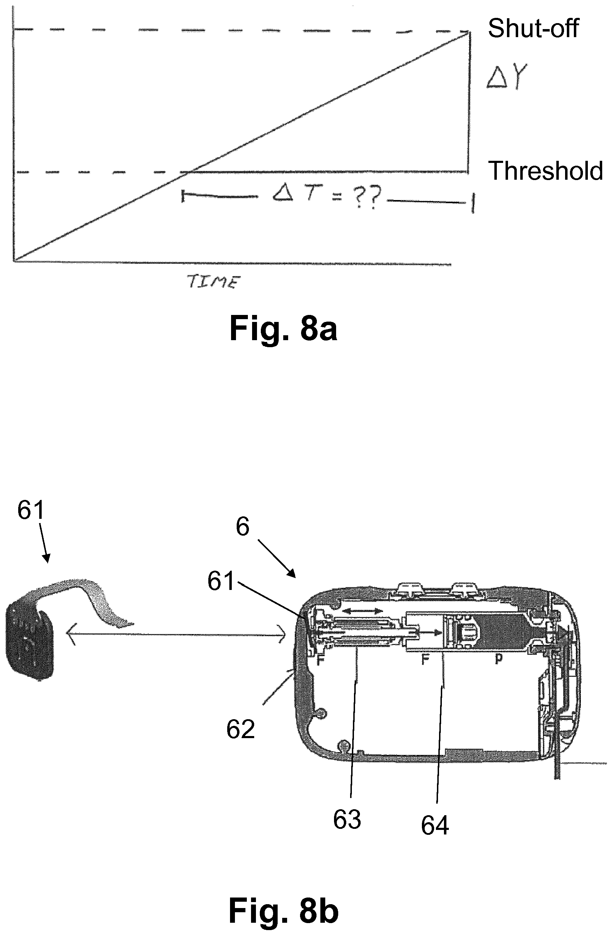

Figure 8a and Figure 8b illustrate the so called "forcesensor technology" widely used in prior art. As illustrated in Figure 8b, a force sensor 61 is installed in an infusion pump 6, for example a durable pump. The infusion pump 6 includes an enclosure 62, a gearing mechanism 63 and an ampule 64. Figure 8a illustrates the force recorded by the force sensor 61 during operation of the infusion pump 6. As illustrated in Figure 8a, a threshold level is defined and shut-off level is defined. The recorded force is compared to the threshold level and the shut-off level in order to control operation of the infusion pump 6.

As illustrated in Figure 8b, the force recorded by the force sensor 61 includes errors, which depend on the stiffness of the enclosure 62, on limitations in the gearing mechanism 63, on the expansion of the ampule 64, etc. Accordingly, precise monitoring of the flow of the fluid of the insulin administration system is not enabled by the analysis of the force signal recorded by the force sensor 61.

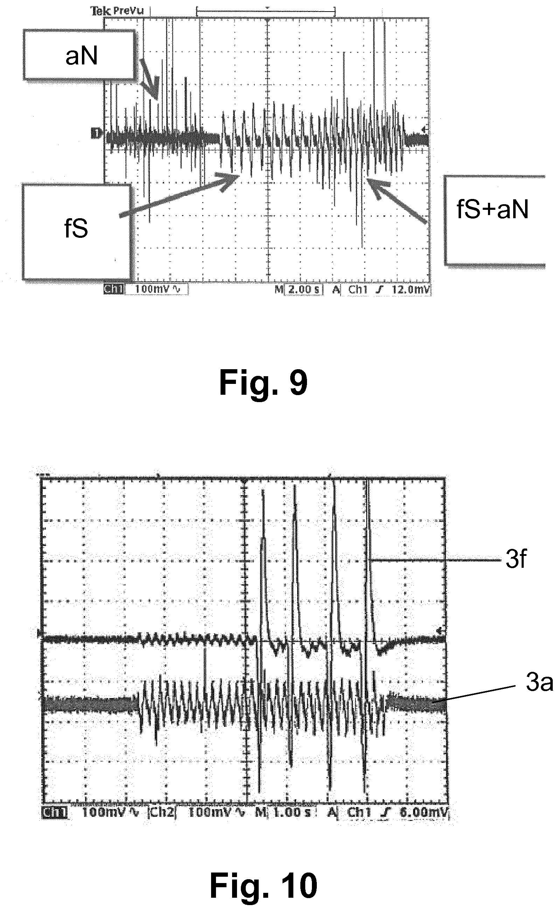

Figure 9 illustrates schematically a signal that was recorded by a single mechanical sensor, for example by a piezo-electric element. The single mechanical sensor is comprised in an insulin administration system for the purpose of monitoring the flow of the fluid. For example, the single mechanical sensor is the first mechanical sensor 13 of the flow sensor 1 illustrated in Figure 1a. As illustrated in Figure 9, on the left, during a first time period, the mechanical sensor records an ambient noise signal aN. During the first time period, the pump was stopped and ambient noise was applied to the mechanical sensor. As illustrated in Figure 9, in the middle, during a second time period, the flow signal fS was recorded.

During the second time period, the pump was activated and no ambient noise was applied to the mechanical sensor. As illustrated in Figure 9, the flow signal fS exhibits a regular pattern corresponding to the bursts of the activated pump. The flow signal fS enables to monitor the fluid administration system sufficiently precisely. As illustrated in Figure 9, on the right, during a third time period, the flow and ambient noise signal fS+aN was recorded. During the third time period, the pump was activated and ambient noise was applied to the mechanical sensor. As illustrated in Figure 9, the flow and ambient noise signal fS+aN is not suitable for precise monitoring of the fluid administration system. In particular, the bursts of the activated pump cannot be identified in the flow and ambient noise signal fS+aN.

The flow sensor 1 according to the invention is designed that the first mechanical sensor 13 records a raw flow signal 3r, whereas the second mechanical sensor 14 records the ambient noise signal 3a. The raw flow signal 3r corresponds the flow and ambient noise signal fS+aN illustrated in the third time period of Figure 9. The ambient noise signal 3a corresponds to the ambient noise signal illustrated in the first time period of Figure 9. The raw flow signal 3r and the ambient noise signal 3a are received in the signal processing unit 3 which is designed to determine the flow signal 3f in such a manner that the flow signal 3f exhibits less ambient noise than the raw flow signal 3r. The signal processing unit 3 is designed that ambient noise can be removed from the raw flow signal 3r at least partly.

Figure 10 illustrates schematically the flow signal 3f which has been determined by a signal processing unit 3 receiving a raw flow signal 3r (not shown in Figure 10) and the ambient noise signal 3a. Each motor burst can be identified clearly in the flow signal 3f. As illustrated in Figure 10, the motor bursts have a regular pattern, which indicates that no occlusion exists in the fluid administration system.

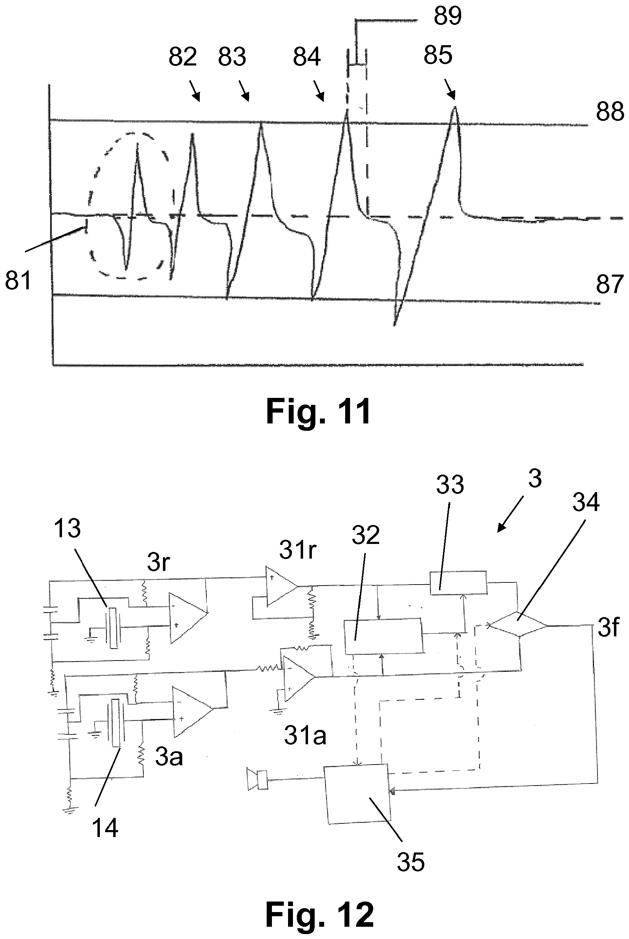

Figure 11 illustrates schematically a sequence of motor bursts 81, 82, 83, 84, 85. The dashed line indicates that the motor burst 81 corresponds to one motor burst. A low threshold 87 and a high threshold 88 is defined. If a motor burst 81, 82, 83, 84, 85 crosses the low threshold 87 or the high threshold 88, the pump can be deactivated in order to prevent damages to the fluid administration system. As indicated with reference sign 89, the fall time of a motor burst can be used to determine if the fluid administration system operates properly. For example, the fall time can be analysed in order to determine if the fluid path is completely free, partially blocked, practically fully blocked, etc.

Figure 12 illustrates schematically an electronic circuit diagram of a signal processing unit 3. The signal processing unit 3 receives a raw flow signal 3r from the first mechanical sensor 13 and an ambient noise 3a signal from the second mechanical sensor 13. Amplifiers 31 r, 31a are arranged for the amplification of the respective signals. A phase detector 32 is arranged for the detection of the phase between the respective signals. A tunable phase shifter 33 is arranged for shifting the phase of the respective signal. A mixing stage 34 is arranged for mixing the respective signals and for determining a flow signal 3f that has less ambient noise than the raw flow signal 3r. A computerized device 35, such as a microprocessor, is arranged that receives the flow signal 3f for further analysis and monitoring of the fluid administration system. As indicated by the dashed lines in Figure 12, the computerized device 35 receives a signal for active phase detection, for active phase control and for active mixing. As illustrated in Figure 12, the computerized device 35 can be connected to a transducer 36 for the purpose of controlling and calibrating the first mechanical sensor 13 and the second mechanical sensor 14.

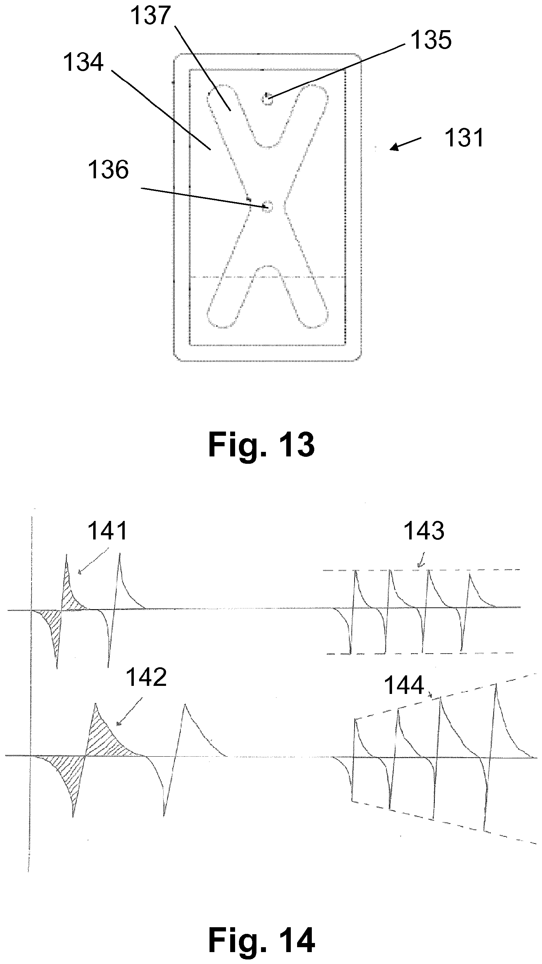

Figure 13 illustrates schematically a container 131 for storing a medical or pharmaceutical liquid disclosed in the application EP2455126, which is included here by reference. The container 131 can be arranged in order to avoid bubbles in the fluid administration system. The container 131 includes a storage compartment 134 for storing the liquid. A inlet opening 135 is arranged for filling the storage compartment 134. An outlet opening 136 is arranged for discharging liquid. A hydrophilic membrane layer 137 is arranged, which is gas-tight in a wet condition and at least covers the outlet opening 136.

Figure 14 illustrates schematically sequences of motor bursts. As illustrated by sequence 141, the rise time and/or the fall time can be sufficiently fast such that it can be determined that no blockage has occurred in the fluid administration system. As illustrated by sequence 142, the rise time and/or the fall time can be significantly slower, indicating that the fluid administration system is partially blocked. As illustrated by sequence 143, the amplitude of the motor bursts can be sufficiently constant from burst to burst, indicating that the fluid administration system is free from a blockage. As illustrated by sequence 144, the amplitude of the motor bursts can increase too quickly, indicating that the fluid administration system is partially or fully blocked.

## Claims

1. A flow sensor (1) for enabling determination of a flow signal corresponding to a flow of a fluid of a fluid administration system, the flow sensor (1) comprising a housing (11), wherein the housing (11) has:
arranged a flow input (1i), a flow chamber (12) and a flow output (1ο) being designed that the flow input (1i) and the flow output (1ο) can be connected to the fluid administration system such that at least an amount of the fluid of the fluid administration system can consecutively pass the flow input (1 i), the flow chamber (12) and the flow output (1ο),
wherein the housing (11) has further arranged a first mechanical sensor (13) for recording a raw flow signal corresponding to the flow of the fluid passing the flow chamber (12) and an ambient noise, wherein the first mechanical sensor (13) can sense mechanical movements induced by a change of the pressure of the fluid in the flow chamber (12), and
wherein the housing (11) has further arranged a second mechanical sensor (14) for recording an ambient noise signal corresponding to the ambient noise, wherein the first mechanical sensor (13) is arranged closer to the flow chamber (12) than the second mechanical sensor (14),
wherein the housing (11) and the arrangement of the first mechanical sensor (13) and the arrangement of the second mechanical sensor (14) are designed that a signal processing unit (3) receiving the raw flow signal (3r) and the ambient noise signal (3a) is enabled to determine the flow signal (3f) from the raw flow signal (3r) and the ambient noise signal (3a) in such a manner that the flow signal (3f) exhibits less ambient noise than the raw flow signal (3r).

2. The flow sensor (1) according to claim 1, wherein the first mechanical sensor (13) is arranged at a first location of the housing (11) and the second mechanical sensor is arranged at a second location of the housing (11), preferably the first mechanical sensor (13) and the second mechanical sensor (14) are arranged at opposite sides of the housing (11).

3. The flow sensor (1) according to one of claims 1 to 3, designed such that the amount of fluid is essentially in direct contact with the first mechanical sensor (13), wherein preferably a thin membrane (132) is arranged for sterilization purposes.

4. The flow sensor (1) according to one of claims 1 to 3, wherein the second mechanical sensor (14) is arranged at a location of the housing (11) providing an open chamber (114) between the second mechanical sensor (14) and the housing (11).

5. The flow sensor (1) according to one of claims 1 to 4, designed such that at least an amount of fluid flows at least once essentially vertically towards the first mechanical sensor (13), at least once essentially vertically away from the first mechanical sensor (13), and/or essentially parallel along the first mechanical sensor (13).

6. The flow sensor (1) according to one of claims 1 to 5, designed such that at least one of width, height, and diameter of at least an amount of fluid flowing through the flow sensor (1) is kept essentially constant.

7. The flow sensor (1) according to one of claims 1 to 6, wherein the flow chamber (12) has essentially the form of a cylinder, the cylinder preferably having a height being defined in the direction of a surface of the housing (11) which is smaller than a diameter of the cylinder, or wherein the flow chamber (12) has essentially the form of a cuboid, the cuboid preferably having a height being defined in the direction of a surface of the housing (11) which is smaller than a width of the cuboid and/or a length of the cuboid.

8. The flow sensor (1) according to one of claims 1 to 7, wherein the first mechanical sensor (13) and/or the second mechanical sensor (14) have essentially a disc-shaped form.

9. The flow sensor (1) according to one of claims 1 to 8, wherein the first mechanical sensor (13) and/or the second mechanical sensor (14) have essentially a rectangularform.

10. The flow sensor (1) according to one of claims 1 to 9, wherein the first mechanical sensor (13) and/or the second mechanical sensor (14) includes a piezoelectric element, preferably a piezoelectric buzzer.

11. The flow sensor (1) according to one of claims 1 to 10, being mounted in one of: a patch pump, a durable pump, an adapter, or an infusion head.

12. The flow sensor (1) according to one of claims 1 to 11, being connected to a signal processing unit (3), the signal processing unit (3) having a first input for receiving the raw flow signal (3r) from the first mechanical sensor (13), and a second input for receiving the ambient noise signal (3a) from the second mechanical sensor (14), the signal processing unit (3) being enabled to determine from the raw flow signal (3r) and the ambient noise signal (3a) the flow signal (3f) in such a manner that the flow signal (3f) exhibits less ambient noise than the raw flow signal (3r).

13. The flow sensor (1) according to claim 12, wherein the signal processing unit (3) is enabled to subtract the ambient noise signal (3a) from the raw flow signal (3r).

14. The flow sensor (1) according to claim 12 or 13, wherein the signal processing unit (3) is enabled to compare a rise time and/or a fall time of the flow signal (3f) and/or an amplitude of several peaks in the flow signal (3f) to a predetermined threshold in order to detect if the fluid administrating system does not or does include a blockage, in particular an occlusion.

## Patentansprüche

1. Strömungssensor (1) zum Ermöglichen der Bestimmung eines Strömungssignals, das einer Strömung eines Fluids eines Fluidverabreichungssystems entspricht, wobei der Strömungssensor (1) ein Gehäuse (11) umfasst, wobei im Gehäuse (11) folgende Elemente angeordnet sind:
ein Strömungseingang (1i), eine Strömungskammer (12) und ein Strömungsausgang (1o), wobei der Strömungseingang (1i) und der Strömungsausgang (1o) derart mit dem Fluidverabreichungssystem verbindbar sind, dass zumindest eine Menge des Fluids des Fluidverabreichungssystems nacheinander den Strömungseingang (1i), die Strömungskammer (12) und den Strömungsausgang (1o) passieren kann,
wobei im Gehäuse (11) ferner ein erster mechanischer Sensor (13) zum Aufnehmen eines Rohströmungssignals angeordnet ist, das der Strömung des die Strömungskammer (12) passierenden Fluids und einem Umgebungsrauschen entspricht, wobei der erste mechanische Sensor (13) mechanische Bewegungen erfassen kann, die durch eine Änderung des Drucks des Fluids in der Strömungsskammer (12) induziert werden, und
wobei im Gehäuse (11) ferner ein zweiter mechanischer Sensor (14) zum Aufnehmen eines dem Umgebungsrauschen entsprechenden Signals angeordnet ist, wobei der erste mechanische Sensor (13) näher an der Strömungskammer (12) angeordnet ist als der zweite mechanische Sensor (14),
wobei das Gehäuse (11) und die Anordnung des ersten mechanischen Sensors (13) und die Anordnung des zweiten mechanischen Sensors (14) so ausgebildet sind, dass eine das Rohströmungssignal (3r) und das Umgebungsrauschsignal (3a) empfangende Signalverarbeitungseinheit (3) in der Lage ist, aus dem Rohströmungssignal (3r) und dem Umgebungsrauschsignal (3a) das Strömungssignal (3f) derart zu bestimmen, dass das Strömungssignal (3f) weniger Umgebungsrauschen zeigt als das Rohströmungssignal (3r).

2. Strömungssensor (1) nach Anspruch 1, wobei der erste mechanische Sensor (13) an einer ersten Stelle des Gehäuses (11) und der zweite mechanische Sensor an einer zweiten Stelle des Gehäuses (11) angeordnet ist, wobei der erste mechanische Sensor (13) und der zweite mechanische Sensor (14) vorzugsweise an gegenüberliegenden Seiten des Gehäuses (11) angeordnet sind.

3. Strömungssensor (1) nach einem der Ansprüche 1 bis 3, derart ausgebildet, dass die Menge an Fluid im Wesentlichen in direktem Kontakt mit dem ersten mechanischen Sensor (13) steht, wobei vorzugsweise eine dünne Membran (132) zur Sterilisation angeordnet ist.

4. Strömungssensor (1) nach einem der Ansprüche 1 bis 3, wobei der zweite mechanische Sensor (14) an einer Stelle des Gehäuses (11) angeordnet ist, die eine offene Kammer (114) zwischen dem zweiten mechanischen Sensor (14) und dem Gehäuse (11) bereitstellt.

5. Strömungssensor (1) nach einem der Ansprüche 1 bis 4, derart ausgebildet, dass zumindest eine Menge an Fluid zumindest einmal im Wesentlichen vertikal zum ersten mechanischen Sensor (13) hin strömt, zumindest einmal im Wesentlichen vertikal vom ersten mechanischen Sensor (13) weg strömt und/oder im Wesentlichen parallel entlang des ersten mechanischen Sensors (13) strömt.

6. Strömungssensor (1) nach einem der Ansprüche 1 bis 5, derart ausgebildet, dass mindestens eines von Breite, Höhe und Durchmesser von mindestens einer den Strömungssensor (1) durchströmenden Menge an Fluid im Wesentlichen konstant gehalten wird.

7. Strömungssensor (1) nach einem der Ansprüche 1 bis 6, wobei die Strömungskammer (12) im Wesentlichen die Form eines Zylinders aufweist, wobei der Zylinder vorzugsweise eine in Richtung einer Oberfläche des Gehäuses (11) definierte Höhe aufweist, die kleiner als ein Durchmesser des Zylinders ist, oder wobei die Strömungskammer (12) im Wesentlichen die Form eines Quaders aufweist, wobei der Quader vorzugsweise eine in Richtung einer Oberfläche des Gehäuses (11) definierte Höhe aufweist, die kleiner als eine Breite des Quaders und/oder eine Länge des Quaders ist.

8. Strömungssensor (1) nach einem der Ansprüche 1 bis 7, wobei der erste mechanische Sensor (13) und/oder der zweite mechanische Sensor (14) im Wesentlichen eine Rundscheibenform aufweisen.

9. Strömungssensor (1) nach einem der Ansprüche 1 bis 8, wobei der erste mechanische Sensor (13) und/oder der zweite mechanische Sensor (14) im Wesentlichen eine rechteckige Form aufweisen.

10. Strömungssensor (1) nach einem der Ansprüche 1 bis 9, wobei der erste mechanische Sensor (13) und/oder der zweite mechanische Sensor (14) ein piezoelektrisches Element, vorzugsweise einen piezoelektrischen Summer, beinhaltet.

11. Strömungssensor (1) nach einem der Ansprüche 1 bis 10, montiert in einer der folgenden Vorrichtungen: einer Patch-Pumpe, einer dauerhaften Pumpe, einem Adapter oder einem Infusionskopf.

12. Strömungssensor (1) nach einem der Ansprüche 1 bis 11, der mit einer Signalverarbeitungseinheit (3) verbunden ist, wobei die Signalverarbeitungseinheit (3) einen ersten Eingang zum Empfangen des Rohströmungssignals (3r) vom ersten mechanischen Sensor (13) und einen zweiten Eingang zum Empfangen des Umgebungsrauschsignals (3a) vom zweiten mechanischen Sensor (14) aufweist, wobei die Signalverarbeitungseinheit (3) in der Lage ist, aus dem Rohströmungssignal (3r) und dem Umgebungsrauschsignal (3a) das Strömungssignal (3f) derart zu bestimmen, dass das Strömungssignal (3f) ein geringeres Umgebungsrauschen zeigt als das Rohströmungssignal (3r).

13. Strömungssensor (1) nach Anspruch 12, wobei die Signalverarbeitungseinheit (3) in der Lage ist, das Umgebungsrauschsignal (3a) vom Rohströmungssignal (3r) zu subtrahieren.

14. Strömungssensor (1) nach Anspruch 12 oder 13, wobei die Signalverarbeitungseinheit (3) in der Lage ist, eine Anstiegszeit und/oder eine Abfallzeit des Strömungssignals (3f) und/oder eine Amplitude mehrerer Peaks im Strömungssignal (3f) mit einem vorbestimmten Schwellenwert zu vergleichen, um zu erkennen, ob das Fluidverabreichungssystem eine Blockade, insbesondere eine Okklusion, beinhaltet oder nicht.

## Revendications

1. Capteur (1) d'écoulement destiné à permettre la détermination d'un signal d'écoulement correspondant à un écoulement d'un fluide d'un système d'administration de fluide, le capteur (1) d'écoulement comportant un boîtier (11), le boîtier (11) :
étant équipé d'une entrée (1i) d'écoulement, d'une chambre (12) d'écoulement et d'une sortie (1o) d'écoulement qui sont conçues de telle façon que l'entrée (1i) d'écoulement et la sortie (1o) d'écoulement puissent être reliées au système d'administration de fluide de telle façon qu'au moins une quantité du fluide du système d'administration de fluide puisse franchir consécutivement l'entrée (1i) d'écoulement, la chambre (12) d'écoulement et la sortie (1o) d'écoulement,
le boîtier (11) étant en outre équipé d'un premier capteur mécanique (13) servant à enregistrer un signal brut d'écoulement correspondant à l'écoulement du fluide franchissant la chambre (12) d'écoulement et un bruit ambiant, le premier capteur mécanique (13) pouvant détecter des mouvements mécaniques induits par une variation de la pression du fluide dans la chambre (12) d'écoulement, et
le boîtier (11) étant en outre équipé d'un second capteur mécanique (14) servant à enregistrer un signal de bruit ambiant correspondant au bruit ambiant, le premier capteur mécanique (13) étant disposé plus près de la chambre (12) d'écoulement que le second capteur mécanique (14),
le boîtier (11) et la disposition du premier capteur mécanique (13) et la disposition du second capteur mécanique (14) étant conçus de telle façon qu'une unité (3) de traitement de signaux recevant le signal brut (3r) d'écoulement et le signal (3a) de bruit ambiant soit rendue apte à déterminer le signal (3f) d'écoulement à partir du signal brut (3r) d'écoulement et du signal (3a) de bruit ambiant de telle manière que le signal (3f) d'écoulement présente moins de bruit ambiant que le signal brut (3r) d'écoulement.

2. Capteur (1) d'écoulement selon la revendication 1, le premier capteur mécanique (13) étant disposé à un premier emplacement du boîtier (11) et le second capteur mécanique étant disposé à un second emplacement du boîtier (11), le premier capteur mécanique (13) et le second capteur mécanique (14) étant de préférence disposés sur des côtés opposés du boîtier (11).

3. Capteur (1) d'écoulement selon l'une des revendications 1 à 3, conçu de telle façon que la quantité de fluide soit essentiellement en contact direct avec le premier capteur mécanique (13), une membrane mince (132) étant de préférence mise en place à des fins de stérilisation.

4. Capteur (1) d'écoulement selon l'une des revendications 1 à 3, le second capteur mécanique (14) étant disposé à un emplacement du boîtier (11) ménageant une chambre ouverte (114) entre le second capteur mécanique (14) et le boîtier (11).

5. Capteur (1) d'écoulement selon l'une des revendications 1 à 4, conçu de telle façon qu'au moins une quantité de fluide s'écoule au moins une fois essentiellement verticalement vers le premier capteur mécanique (13), au moins une fois essentiellement verticalement en s'écartant du premier capteur mécanique (13), et/ou essentiellement parallèlement le long du premier capteur mécanique (13).

6. Capteur (1) d'écoulement selon l'une des revendications 1 à 5, conçu de telle façon qu'au moins une dimension parmi une largeur, une hauteur et un diamètre d'au moins une quantité de fluide s'écoulant à travers le capteur (1) d'écoulement soit maintenu essentiellement constante.

7. Capteur (1) d'écoulement selon l'une des revendications 1 à 6, la chambre (12) d'écoulement présentant essentiellement la forme d'un cylindre, le cylindre présentant de préférence une hauteur, définie dans la direction d'une surface du boîtier (11), qui est plus petite qu'un diamètre du cylindre, ou la chambre (12) d'écoulement présentant essentiellement la forme d'un parallélépipède, le parallélépipède présentant de préférence une hauteur, définie dans la direction d'une surface du boîtier (11), qui est plus petite qu'une largeur du parallélépipède et/ou une longueur du parallélépipède.

8. Capteur (1) d'écoulement selon l'une des revendications 1 à 7, le premier capteur mécanique (13) et/ou le second capteur mécanique (14) présentant essentiellement une forme en disque.

9. Capteur (1) d'écoulement selon l'une des revendications 1 à 8, le premier capteur mécanique (13) et/ou le second capteur mécanique (14) présentant essentiellement une forme rectangulaire.

10. Capteur (1) d'écoulement selon l'une des revendications 1 à 9, le premier capteur mécanique (13) et/ou le second capteur mécanique (14) comprenant un élément piézoélectrique, de préférence un vibreur piézoélectrique.

11. Capteur (1) d'écoulement selon l'une des revendications 1 à 10, celui-ci étant monté dans un dispositif parmi : une pompe pour timbre, une pompe durable, un adaptateur, et une tête de perfusion.

12. Capteur (1) d'écoulement selon l'une des revendications 1 à 11, celui-ci étant relié à une unité (3) de traitement de signaux, l'unité (3) de traitement de signaux possédant une première entrée destinée à recevoir le signal brut (3r) d'écoulement en provenance du premier capteur mécanique (13) et une seconde entrée destinée à recevoir le signal (3a) de bruit ambiant en provenance du second capteur mécanique (14), l'unité (3) de traitement de signaux étant rendue apte à déterminer, à partir du signal brut (3r) d'écoulement et du signal (3a) de bruit ambiant, le signal (3f) d'écoulement de telle manière que le signal (3f) d'écoulement présente moins de bruit ambiant que le signal brut (3r) d'écoulement.

13. Capteur (1) d'écoulement selon la revendication 12, l'unité (3) de traitement de signaux étant rendue apte à soustraire le signal (3a) de bruit ambiant du signal brut (3r) d'écoulement.

14. Capteur (1) d'écoulement selon la revendication 12 ou 13, l'unité (3) de traitement de signaux étant rendue apte à comparer un temps de montée et/ou un temps de baisse du signal (3f) d'écoulement et/ou une amplitude de plusieurs pics dans le signal (3f) d'écoulement à un seuil prédéterminé afin de détecter si le système d'administration de fluide inclut ou non un blocage, en particulier une occlusion.
